(19) Europäisches Patentamt
European Patent Office
Office européen des brevets

(11) Veröffentlichungsnummer: **0 042 089**
**A2**

# (12) EUROPÄISCHE PATENTANMELDUNG

(21) Anmeldenummer: **81104159.9**

(22) Anmeldetag: **01.06.81**

(51) Int. Cl.³: **C 07 D 417/04**, C 07 D 401/04, A 61 K 31/435

(30) Priorität: **12.06.80 DE 3022030**

(43) Veröffentlichungstag der Anmeldung: **23.12.81**
**Patentblatt 81/51**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Materne, Carsten, Dr., Hans-Boeckler-Strasse 31, D-5300 Bonn 3 (DE)**

## (54) 4-Heteroring-substituierte Dihydropyridine, Verfahren zu ihrer Herstellung, ihre Verwendung in Arzneimitteln sowie deren Herstellung.

(57) Die vorliegende Erfindung betrifft neue 4-Heteroring-substituierte Dihydropyridine, mehrere Verfahren zu ihrer Herstellung, ihre Verwendung als Arzneimittel, insbesondere als kreislaufbeeinflussende Arzneimittel, vorzugsweise als blutdrucksenkende und coronaraktive Arzneimittel sowie Verfahren zur Herstellung dieser Arzneimittel.

EP 0 042 089 A2

ACTORUM AG

BAYER AKTIENGESELLSCHAFT
5090 Leverkusen, Bayerwerk
Zentralbereich
Patente, Marken und Lizenzen
KS/bc/c
Ib (Pha)

# 4-Heteroring-substituierte Dihydropyridine, Verfahren zu ihrer Herstellung, ihre Verwendung in Arzneimitteln sowie deren Herstellung

Vorliegende Erfindung betrifft neue 4-Heteroring-substituierte Dihydropyridine, mehrere Verfahren zu ihrer Herstellung, ihre Verwendung als Arzneimittel, insbesondere als kreislaufbeeinflussende Arzneimittel, vorzugsweise als blutdrucksenkende und coronaraktive Arzneimittel sowie Verfahren zur Herstellung dieser Arzneimittel.

Es ist bereits bekannt, daß 1,4-Dihydropyridinderivate Kreislauf-beeinflussende Eigenschaften aufweisen. So ist z.B. der 2,6-Dimethyl-4-(2'-nitrophenyl)-1,4-dihydropyridin-3,5-dicarbonsäuredimethylester (Nifedipin, vgl. DT-PS 1 607 827) als Verbindung bekannt, die coronarvasodilatierende Wirkung besitzt.

Durch Heterocyclen der Imidazol- bzw. Thiazolreihe substituierte 1,4-Dihydropyridine sind bisher nicht beschrieben. Die Erfindung betrifft daher neue 1,4-Dihydropyridine der allgemeinen Formel (I)

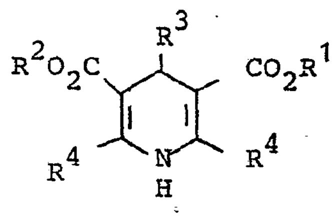

(I)

in welcher

$R^3$ steht für Pyrazolyl, Imidazolyl, Oxazolyl, Isoxazolyl, Thiazolyl, Isothiazolyl, Triazolyl, Oxadiazolyl, Thiadiazolyl, Pyrazidnyl, Oxazinyl, Thiazinyl, Indolizinyl, Indolyl, Benzofuranyl, Benzothienyl, Indazolyl, Benzimidazolyl, Benzoxazolyl, Benzisoxazolyl, Benzthiazolyl, Benzisothiazolyl, Benztriazolyl, Benzoxadiazolyl, Benzthiadiazolyl, Cinnolinyl, Chinoxazinyl, Chinazolinyl, Phthalazinyl, Naphthyridinyl oder Benzotriazinyl, wobei die genannten Heterocyclen gegebenenfalls 1, 2 oder 3 gleiche oder verschiedene Substituenten aus der Gruppe Phenyl, Alkyl, Alkoxy, Alkylen, Dioxyalkylen, Halogen, Trifluormethyl, Trifluormethoxy, Amino, Alkylamino, Nitro, Cyano, Azido, Carbonamido, Sulfonamido oder $SO_m$-Alkyl (m = 0 oder 2) enthalten,

$R^4$ für Wasserstoff, einen geradkettigen oder verzweigten Alkylrest, einen Arylrest oder einen Aralkylrest steht und

$R^1$ und $R^2$ gleich oder verschieden sind und jeweils für einen geradkettigen, verzweigten oder cyclischen, gesättigten oder ungesättigten Kohlenwasserstoffrest stehen, der durch ein Sauerstoffatom in der Kette unterbrochen sein kann, und/oder der gegebenenfalls substituiert ist durch Halogen, Pyridyl,

Phenyl oder Phenoxy, wobei die Phenylgruppen ihrerseits substituiert sein können durch Halogen, Cyano, Dialkylamino, Alkoxy, Alkyl, Trifluormethyl oder Nitro, oder wobei der Kohlenwasserstoffrest substituiert sein kann durch eine Aminogruppe, wobei diese Aminogruppe durch zwei gleiche oder verschiedene Substituenten aus der Gruppe Alkyl, Alkoxyalkyl, Aryl oder Aralkyl substituiert ist, oder wobei die Aminogruppe in der Weise substituiert ist, daß 2 Substituenten gemeinsam mit dem Stickstoffatom einen 5- bis 7-gliedrigen Ring bilden, der als weiteres Heteroatom Sauerstoff oder Schwefel oder eine N-Alkyl-Gruppierung enthalten kann;

sowie gegebenenfalls ihre pharmazeutisch unbedenklichen Salze und ihre diastereomeren Gemische, Racemformen sowie Antipoden der Verbindungen der allgemeinen Formel I.

Die Racemformen lassen sich ebenso wie die Diastereomeren nach bekannten Methoden in stereoisomer einheitliche Bestandteile trennen (vergl. z. B. E. Eliel, Stereochemistry of Carbon Compounds, McGraw Hill, 1962).

Die neuen Verbindungen besitzen Kreislauf-beeinflussende Eigenschaften, insbesondere erweitern sie die Coronargefäße und senken den Blutdruck.

Vorzugsweise seien genannt Verbindungen der allgemeinen Formel (I), in welcher

$R^3$ steht für Pyrazolyl, Imidazolyl, Oxazolyl, Isoxazolyl, Thiazolyl, Triazolyl, Oxadiazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzisoxazolyl, Benzthiazolyl, Benztriazolyl, Benzoxadiazolyl, Cinnolinyl, Phthalazinyl, Naphthyridinyl oder Benzotriazinyl, wobei die genannten Heterocyclen gegebenenfalls 1, 2 oder 3 gleiche oder verschiedene Substituenten aus der Gruppe Phenyl, Alkyl mit 1 bis 4 C-Atomen, Alkoxy mit 1 bis 4 C-Atomen, Alkylen mit

3 bis 5 C-Atomen, Dioxyalkylen mit 1 bis 3 C-Atomen, Fluor, Chlor, Brom, Trifluormethyl, Trifluormethoxy, Alkylamino mit je 1 oder 2 Alkylgruppen, die jeweils 1 bis 4 Kohlenstoffatome enthalten, Nitro, Cyano, Azido, Carbonamido, Sulfonamido, Amino oder $SO_m$-Alkyl (m = 0 oder 2), wobei Alkyl 1 bis 4 Kohlenstoffatome enthalten,

$R^4$ für Wasserstoff, einen geradkettigen oder verzweigten Alkylrest mit 1 bis 4 Kohlenstoffatomen, einen Phenylrest oder einen Benzylrest steht und

$R^1$ und $R^2$ gleich oder verschieden sind und jeweils für einen geradkettigen, verzweigten oder cyclischen gesättigten oder ungesättigten Kohlenwasserstoffrest mit 1 bis 8 Kohlenstoffatomen stehen, der durch ein Sauerstoffatom in der Kette unterbrochen sein kann und/oder der gegebenenfalls substituiert ist durch Fluor, Chlor, Pyridyl, Phenyl oder Phenoxy, wobei die Phenylgruppen ihrerseits substituiert sein können durch Fluor, Chlor, Brom, Cyano, Trifluormethyl, Nitro, Dialkylamino mit 1 bis 4 Kohlenstoffatomen je Alkylrest, Alkoxy mit 1 bis 4 Kohlenstoffatomen oder Alkyl mit 1 bis 4 Kohlenstoffatomen oder

wobei der Kohlenwasserstoffrest substituiert sein kann durch eine Aminogruppe, die ihrerseits durch 2 gleiche oder verschiedene Substituenten aus der Gruppe Alkyl mit 1 bis 4 C-Atomen, Alkoxyalkyl mit 2 bis 6 C-Atomen, Phenyl, Benzyl oder Phenethyl substituiert ist, oder

wobei die Aminogruppe in der Weise substituiert ist,

daß zwei Substituenten gemeinsam mit dem Stickstoffatom einen 5- bis 7-gliedrigen Ring bilden, der als weiteres Heteroatom Sauerstoff oder eine N-Methylgruppierung enthalten kann.

Insbesondere seien Verbindungen der allgemeinen Formel (I) genannt, in welcher

$R^3$ für einen 5-gliedrigen Heterocyclus aus der Imidazol- oder Thiazolreihe steht, der gegebenenfalls durch Alkyl mit 1 bis 4 Kohlenstoffatomen oder Phenyl substituiert ist,

$R^4$ für Methyl, Ethyl, Phenyl oder Benzyl steht und

$R^1$ und $R^2$ gleich oder verschieden sind und für einen geradkettigen oder verzweigten gesättigten oder ungesättigten Kohlenwasserstoffrest mit bis zu 6 Kohlenstoffatomen stehen, der gegebenenfalls durch ein Sauerstoffatom in der Kette unterbrochen ist und/oder der gegebenenfalls substituiert ist durch Fluor oder Chlor.

Die Herstellung der neuen Verbindungen kann in an sich bekannter Weise dadurch erfolgen, daß man

A) Aldehyde der Formel (II)

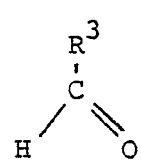

(II)

in der

$R^3$ die oben angegebene Bedeutung besitzt,

mit $NH_3$ und mit Acetessigsäureestern der Formel (III)

$$R^4-\overset{\overset{O}{\|}}{C}-CH_2-\overset{\overset{O}{\|}}{C}-OR^1 \qquad \text{(III)}$$

in der
$R^1$ und $R^2$ die oben angegebene Bedeutung besitzen,

oder mit Enaminoestern der Formel (IV)

$$R^4-\underset{\underset{NH_2}{|}}{C}=CH-\overset{\overset{O}{\|}}{C}-OR^1 \qquad \text{(IV)}$$

in der
$R^1$ und $R^4$ die oben angegebene Bedeutung besitzen,

in einem organischen Lösungsmittel umsetzt, wobei nach dieser Verfahrensvariante Verbindungen der Formel (I) mit symmetrischen Estergruppen ($R^1 = R^2$) erhalten werden, oder

B) Ylidenverbindungen der Formel (V)

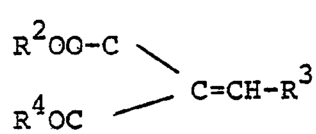

(V)

in der
$R^2$,$R^3$ und $R^4$ die oben angegebene Bedeutung besitzen,

mit Enaminoestern der Formel (IV) in einem organischen inerten Lösungsmittel umsetzt.

Als organisches Lösungsmittel kann Alkohol, Dioxan, Essigester, Eisessig, Dimethylformamid oder Acetonitril genommen werden. Die Reaktionstemperaturen können in einem Bereich zwischen 20 und 150°C variiert werden. Vorzugsweise arbeitet man bei der Siedetemperatur des Lösungsmittels.

Die verwendbaren Ausgangsstoffe sind meistens bekannte Verbindungen oder sie können nach bekannten Verfahren hergestellt werden.

Als Beispiele für Aldehyde der Formel (II) seien genannt: Thiazol-(2)-aldehyd, 2-Phenylthiazol-(4)-aldehyd, 2-Methylthiazol-(4)-aldehyd, 1-Methylimidazol-2-aldehyd.

Als Acetessigsäureester eignen sich für diese Umsetzung z.B.: Acetessigsäuremethylester, Acetessigsäureethylester, Acetessigsäurepropylester, Acetessigsäureisopropylester, Acetessigsäurebutylester, Acetessigsäuresec.-Butylester, Acetessigsäureisobutylester.

Die für die Umsetzung geeigneten Enaminoester der Formel (IV) erhält man aus den entsprechenden Acetessigestern der Formel (III) durch Umsetzen mit Ammoniak.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-,Milch- und Traubenzucker), Emulgiermittel, wie nicht-ionogene oder anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den o.g. Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg vorzugsweise etwa 0,05 bis 5 mg/kg Körpergewicht pro Tag zur Erzielung wirksamer Ergebnisse zu verabreichen und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Im folgenden sei die Erfindung anhand einiger Beispiele zur Herstellung der neuen Dihydropyridin-Derivate erläutert:

Beispiel 1

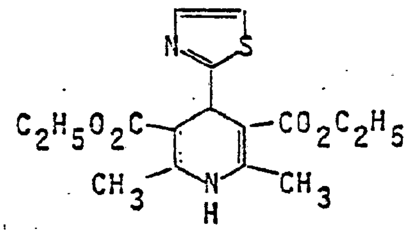

a) 2,6-Dimethyl-4-thiazolyl-(2)-1,4-dihydropyridin-3,5-dicarbonsäurediethylester

18 g 2-Formylthiazol werden zusammen mit 41,5 g Acetessigsäureethylester und 12 ml Ammoniak (33 %ig) in 100 $cm^3$ Ethanol 4 bis 5 Stunden unter Rückfluß erhitzt. Man engt ein, läßt abkühlen und kristallisiert den Niederschlag aus Ethanol um.
Ausbeute: 20 g (40 %);
Schmelzpunkt: 184°C bis 186°C.

In analoger Weise werden erhalten:

b) 2,6-Dimethyl-4-thiazolyl-(2)-1,4-dihydropyridin-3,5-dicarbonsäuredimethylester
c) 2,6-Dimethyl-4-thiazolyl-(2)-1,4-dihydropyridin-3,5-dicarbonsäurediisopropylester
d) 2,6-Dimethyl-4-thiazolyl-(2)-1,4-dihydropyridin-3,5-dicarbonsäuredibutylester
e) 2,6-Dimethyl-4-thiazolyl-(2)-1,4-dihydropyridin-3,5-dicarbonsäuredipropylester
f) 2,6-Dimethyl-4-thiazolyl-(2)-1,4-dihydropyridin-3,5-dicarbonsäurediisobutylester.

Beispiel 2

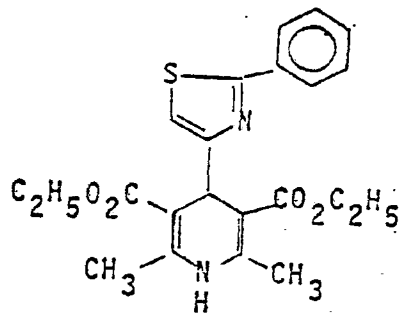

a) 2,6-Dimethyl-4-(2'-phenylthiazolyl-4)-1,4-dihydropyridin-3,5-dicarbonsäurediethylester

18,9 g 2-Phenyl-4-formylthiazol werden mit 25,8 g ß-Aminocrotonsäureethylester in 100 $cm^3$ Ethanol 8 Stunden unter Rückfluß erhitzt. Man engt ein, läßt abkühlen und kristallisiert den Rückstand aus Ethanol um.
Ausbeute: 28,8 g (70 %);
Schmelzpunkt: 160°C.

In analoger Weise erhält man:

b) 2,6-Dimethyl-4-(2'-phenylthiazolyl-(4))-1,4-dihydropyridin-3,5-dicarbonsäuredimethylester

c) 2,6-Dimethyl-4-(2'-phenylthiazolyl-(4))-1,4-dihydropyridin-3,5-dicarbonsäurediisopropylester

d) 2,6-Dimethyl-4-(2'-phenylthiazolyl-(4))-1,4-dihydropyridin-3,5-dicarbonsäuredibutylester.

Beispiel 3

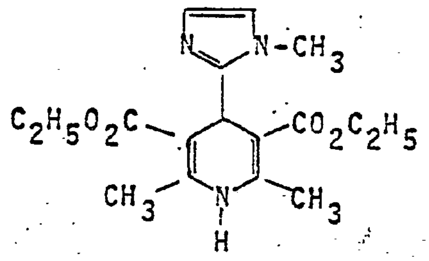

a) 2,6-Dimethyl-4-(1-methylimidazolyl-(2))-1,4-dihydropyridin-3,5-dicarbonsäurediethylester

11 g 1-Methyl-2-formylimidazol und 25,8 g ß-Aminocrotonsäureethylester werden in 100 $cm^3$ Ethanol 4 Stunden unter Rückfluß erhitzt. Nach dem Abkühlen wird abgesaugt und aus Ethanol umkristallisiert.
Ausbeute: 10 g (30 %);
Schmelzpunkt: 223 bis 227°C.

In analoger Weise erhält man:

b) 2,6-Dimethyl-4-(1-methylimidazolyl-(2))-1,4-dihydropyridin-3,5-dicarbonsäuremethylester.

Beispiel 4

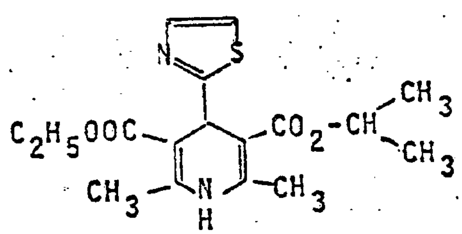

a) 2,6-Dimethyl-4-thiazolyl-(2)-1,4-dihydropyridin-3,5-dicarbonsäure-3-ethylester-5-isopropylester

24,1 g $\chi$-Acetyl-ß-2-thiazolylacrylsäureethylester und 14,3 g ß-Aminocrotonsäureisopropylester werden 3 Stunden in 100 $cm^3$ Ethanol unter Rückfluß erhitzt. Nach dem Erkalten kristallisiert das Produkt aus. Zur Reinigung wird aus Ethanol umkristallisiert.
Ausbeute: 14 g (40 %);
Schmelzpunkt: 165 bis 168°C.

In analoger Weise erhält man:

b) 2,6-Dimethyl-4-thiazolyl-(2)-1,4-dihydropyridin-3,5-dicarbonsäure-3-ethylester-5-methylester

c) 2,6-Dimethyl-4-thiazolyl-(2)-1,4-dihydropyridin-3,5-dicarbonsäure-3-ethylester-5-propylester.

## Patentansprüche

1) 1,4-Dihydropyridine der Formel (I)

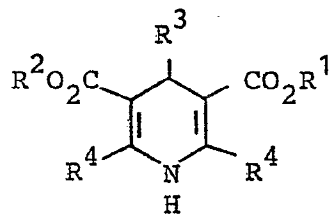

(I)

in welcher

$R^3$ steht für Pyrazolyl, Imidazolyl, Oxazolyl, Isoxazolyl, Thiazolyl, Isothiazolyl, Triazolyl, Oxadiazolyl, Thiadiazolyl, Pyrazinyl, Oxazinyl, Thiazinyl, Indolizinyl, Indolyl, Benzofuranyl, Benzothienyl, Indazolyl, Benzimidazolyl, Benzoxazolyl, Benzisoxazolyl, Benzthiazolyl, Benzisothiazolyl, Benztriazolyl, Benzoxadiazolyl, Benzthiadiazolyl, Cinnolinyl, Chinoxazinyl, Chinazolinyl, Phthalazinyl, Naphthyridinyl oder Benzotriazinyl, wobei die genannten Heterocyclen gegebenenfalls 1, 2 oder 3 gleiche oder verschiedene Substituenten aus der Gruppe Phenyl, Alkyl, Alkoxy, Alkylen, Dioxyalkylen, Halogen, Trifluormethyl, Trifluormethoxy, Amino, Alkylamino, Nitro, Cyano, Azido, Carbonamido, Sulfonamido oder $SO_m$-Alkyl (m = 0 oder 2) enthalten,

$R^4$ für Wasserstoff, einen geradkettigen oder verzweigten Alkylrest, einen Arylrest oder einen Aralkylrest steht und

$R^1$ und $R^2$ gleich oder verschieden sind und jeweils für einen geradkettigen, verzweigten oder cyclischen, gesättigten oder ungesättigten Kohlenwasserstoffrest stehen, der durch ein Sauerstoffatom in der Kette unterbrochen sein kann, und/oder der gegebenenfalls substituiert ist durch Halogen, Pyridyl,

Phenyl oder Phenoxy, wobei die Phenylgruppen ihrerseits substituiert sein können durch Halogen, Cyano, Dialkylamino, Alkoxy, Alkyl, Trifluormethyl oder Nitro, oder wobei der Kohlenwasserstoffrest substituiert sein kann durch eine Aminogruppe, wobei diese Aminogruppe durch zwei gleiche oder verschiedene Substituenten aus der Gruppe Alkyl, Alkoxyalkyl, Aryl oder Aralkyl substituiert ist, oder wobei die Aminogruppe in der Weise substituiert ist, daß 2 Substituenten gemeinsam mit dem Stickstoffatom einen 5- bis 7-gliedrigen Ring bilden, der als weiteres Heteroatom Sauerstoff oder Schwefel oder eine N-Alkyl-Gruppierung enthalten kann,

sowie gegebenenfalls ihre pharmazeutisch unbedenklichen Salze und ihre diastereomeren Gemische, Racemformen sowie Antipoden der Verbindungen der allgemeinen Formel I.

2) 1,4-Dihydropyridine der allgemeinen Formel (I) gemäß Anspruch 1, in welcher

$R^3$ steht für Pyrazolyl, Imidazolyl, Oxazolyl, Isoxazolyl, Thiazolyl, Triazolyl, Oxadiazolyl, Pyrazinyl, Oxazinyl, Thiazinyl, Indolizinyl, Indolyl,

Benzofuranyl, Indazolyl, Benzothienyl, Benzimidazolyl, Benzoxazolyl, Benzisoxazolyl, Benzthiazolyl, Benztriazolyl, Benzoxadiazolyl, Cinnolinyl, Phthalazinyl, Naphthyridinyl oder Benzotriazinyl, wobei die genannten Heterocyclen gegebenenfalls 1, 2 oder 3 gleiche oder verschiedene Substituenten aus der Gruppe Phenyl, Alkyl mit 1 bis 4 C-Atomen, Alkoxy mit 1 bis 4 C-Atomen, Alkylen mit 3 bis 5 C-Atomen, Dioxyalkylen mit 1 bis 3 C-Atomen, Fluor, Chlor, Brom, Trifluormethyl, Trifluormethoxy, Alkylamino mit je 1 oder 2 Alkylgruppen, die jeweils 1 bis 4 Kohlenstoffatome enthalten, Nitro, Cyano, Azido, Carbonamido, Sulfonamido, Amino oder $SO_m$-Alkyl (m = 0 oder 2), wobei Alkyl 1 bis 4 Kohlenstoffatome enthalten,

$R^4$ für Wasserstoff, einen geradkettigen oder verzweigten Alkylrest mit 1 bis 4 Kohlenstoffatomen, einen Phenylrest oder einen Benzylrest steht und

$R^1$ und $R^2$ gleich oder verschieden sind und jeweils für einen geradkettigen, verzweigten oder cyclischen gesättigten oder ungesättigten Kohlenwasserstoffrest mit 1 bis 8 Kohlenstoffatomen stehen, der durch ein Sauerstoffatom in der Kette unterbrochen sein kann und/oder der gegebenenfalls substituiert ist durch Fluor, Chlor, Pyridyl, Phenyl oder Phenoxy, wobei die Phenylgruppen ihrerseits substituiert sein können durch Fluor, Chlor, Brom, Cyano, Trifluormethyl, Nitro, Dialkylamino mit 1 bis 4 Kohlenstoffatomen je Alkylrest, Alkoxy mit 1 bis 4 Kohlenstoffatomen oder Alkyl mit 1

4 Kohlenstoffatomen oder

wobei der Kohlenwasserstoffrest substituiert sein kann durch eine Aminogruppe, die ihrerseits durch 2 gleiche oder verschiedene Substituenten aus der Gruppe Alkyl mit 1 bis 4 C-Atomen, Alkoxyalkyl mit 2 bis 6 C-Atomen, Phenyl, Benzyl oder Phenethyl substituiert ist, oder

wobei die Aminogruppe in der Weise substituiert ist, daß zwei Substituenten gemeinsam mit dem Stickstoffatom einen 5- bis 7-gliedrigen Ring bilden, der als weiteres Heteroatom Sauerstoff oder eine N-Methylgruppierung enthalten kann.

3) Dihydropyridine der allgemeinen Formel (I) gemäß Anspruch 1, in welcher

$R^3$ für einen 5-gliedrigen Heterocyclus aus der Imidazol- oder Thiazolreihe steht, der gegebenenfalls durch Alkyl mit 1 bis 4 Kohlenstoffatomen oder Phenyl substituiert ist,

$R^4$ für Methyl, Ethyl, Phenyl oder Benzyl steht und

$R^1$ und $R^2$ gleich oder verschieden sind und für einen geradkettigen oder verzweigten gesättigten oder ungesättigten Kohlenwasserstoffrest mit bis zu 6 Kohlenstoffatomen stehen, der gegebenenfalls durch ein Sauerstoffatom in der Kette unterbrochen

ist und/oder der gegebenenfalls substituiert ist durch Fluor oder Chlor.

4) Verfahren zur Herstellung der 1,4-Dihydropyridin-Derivate nach Anspruch 1, dadurch gekennzeichnet, daß man in an sich bekannter Weise

A) Aldehyde der Formel (II)

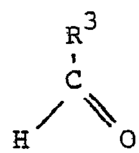

(II)

in der
$R^3$ die oben angegebene Bedeutung besitzt,

mit $NH_3$ und mit Acetessigsäureestern der Formel (III)

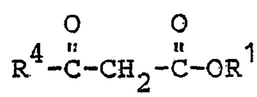

(III)

in der
$R^1$ und $R^1$ die oben angegebene Bedeutung besitzen,

oder mit Enaminoestern der Formel (IV)

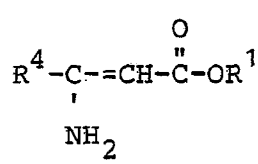

(IV)

in der

$R^1$ und $R^4$ die oben angegebene Bedeutung besitzen,

in einem organischen Lösungsmittel umsetzt, wobei nach dieser Verfahrensvariante Verbindungen der Formel (I) mit symmetrischen Estergruppen ($R^1$ = $R^2$) erhalten werden, oder

B) Ylidenverbindungen der Formel (V)

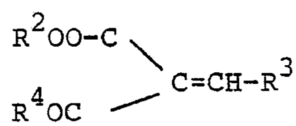

(V)

in der
$R^2$, $R^3$ und $R^4$ die oben angegebene Bedeutung besitzen,

mit Enaminoestern der Formel (IV) in einem organischen inerten Lösungsmittel umsetzt.

5) Verfahren gemäß Anspruch 4, dadurch gekennzeichnet, daß man die Reaktion bei Temperaturen zwischen 20 und 120°C durchführt.

6) Arzneimittel, enthaltend mindestens ein 1,4-Dihydropyridin gemäß Anspruch 1.

7) Arzneimittel mit kreislaufbeeinflussender Wirkung enthaltend mindestens 1 1,4-Dihydropyridin gemäß Anspruch 1.

8) Verfahren zur Herstellung von Arzneimitteln, dadurch gekennzeichnet, daß man mindestens 1 1,4-Dihydropyridin gemäß Anspruch 1, gegebenenfalls

unter Verwendung von üblichen Hilfs- und Trägerstoffen, in eine geeignete Applikationsform überführt.

9) Verwendung von 1,4-Dihydropyridinen gemäß Anspruch 1 bei der Bekämpfung von Erkrankungen.

10) Verwendung von 1,4-Dihydropyridinen gemäß Anspruch 1 bei der Bekämpfung von Kreislauferkrankungen.